Europäisches Patentamt

European Patent Office

Office européen des brevets

(19)

(11) Veröffentlichungsnummer: **0 496 723 B1**

(12) **EUROPÄISCHE PATENTSCHRIFT**

(45) Veröffentlichungstag der Patentschrift: **20.09.95**

(51) Int. Cl.⁶: **G01N 33/86**, C12Q 1/56

(21) Anmeldenummer: **92890015.8**

(22) Anmeldetag: **17.01.92**

(54) **Reagens zur Bestimmung von Faktor VIII-Aktivität.**

(30) Priorität: **25.01.91 AT 164/91**

(43) Veröffentlichungstag der Anmeldung:
**29.07.92 Patentblatt 92/31**

(45) Bekanntmachung des Hinweises auf die
Patenterteilung:
**20.09.95 Patentblatt 95/38**

(84) Benannte Vertragsstaaten:
**AT BE CH DE DK ES FR GB IT LI NL SE**

(56) Entgegenhaltungen:
**EP-A- 0 159 311**

**HAEMOSTASIS, Band 17, Nr. 1-2, 1987, Basel
(CH); DIEIJEN et al., Seiten 14-24**

**ARCHIVES OF BIOCHEMISTRY & BIOPHY-
SICS, Band 268, Nr. 2, 01. Februar 1989, New
York, NY (US); BEALS et al., Seiten 485-501**

**HAEMOSTASIS, Band 19, 1989, Basel (CH);
WAGENVOORD et al., Seiten 196-204**

**THROMBOSIS RESEARCH, Band 27, 1982,
New York, NY (US); GRIFFITH et al., Seiten
289-301**

**HAEMOSTASIS, Band 16, Suppl. 5, 1986, Basel (CH); VAN DE WAART et al., Seite 72**

(73) Patentinhaber: **IMMUNO Aktiengesellschaft
Industriestrasse 67
A-1221 Wien (AT)**

(72) Erfinder: **Lang, Hartmut, Dr.
Färbermühlgasse 13
A-1230 Wien (AT)**
Erfinder: **Moritz, Berta, Dr.
Beatrixgasse 20
A-1030 Wien (AT)**
Erfinder: **Oberreither, Manfred, Dr.
Paradisgasse 61
A-1190 Wien (AT)**
Erfinder: **Lukas, Olga
Lenkgasse 1-3
A-1220 Wien (AT)**

(74) Vertreter: **Wolfram, Gustav, Dipl.-Ing. et al
Patentanwälte
Sonn, Pawloy, Weinzinger & Wolfram,
Riemergasse 14
A-1010 Wien (AT)**

**Beschreibung**

Die Erfindung betrifft ein Reagens zur Bestimmung von Faktor VIII-Aktivität, sowie ein Verfahren zur Bestimmung von Faktor VIII-Aktivität in einer Probe.

Eine Bestimmung von Faktor VIII-Aktivität wurde von R.J. Wagenvoord et al. für den klinischen Gebrauch beschrieben (Haemostasis 19, 196-204 (1989)). Gemäß dieser Methode wird der Faktor VIII-hältigen Probe als Reagens 1 eine Mischung aus Faktor IXa, Thrombin, Calciumionen und Phospholipiden zugegeben, wodurch der zu bestimmende Faktor VIII zunächst aktiviert wird. Der aktivierte Faktor VIII bildet in der Folge mit dem Faktor IXa, den Phospholipiden und den Calciumionen einen Komplex, der in der Lage ist, Faktor X, der in der Folge als weiteres Reagens (Reagens 2) zugegeben werden muß, zu aktivieren. Mit dem aktivierten Faktor X kann in bekannter Weise chromogenes Substrat gespalten werden, wobei aus der Menge der gebildeten photometrisch bestimmbaren Substanz die Faktor VIII-Aktivität der zu untersuchenden Probe rückgerechnet werden kann. Der im komplexbildenden Reagens enthaltene Faktor IXa und das enthaltene Thrombin, sowie der zur Bildung von aktiviertem Faktor Xa verwendete Faktor X sind bovinen Ursprungs.

Ganz analog zu dem genannten Verfahren wird ein kommerziell erhältlicher Test ("Dade[R] Faktor VIII Chromogen"; Hersteller: Fa. Baxter) durchgeführt; auch hier muß Faktor X der Probe in einem eigenen Pipettierschritt zugegeben werden. Ein Nachteil dieser Bestimmungsmethode ist darin zu sehen, daß sie aufgrund der mehrfachen Pipettierungsschritte relativ aufwendig durchzuführen ist. Dieser Nachteil kommt bei Reihenuntersuchungen besonders zum Tragen, da für die Arbeitsvorgänge nur wenig Zeit bleibt (Inkubationszeit: etwa 90 Sekunden; Meßzeit: etwa 60 Sekunden).

Die Zugabe von Faktor X in einem eigenen Pipettierschritt ist deshalb erforderlich, da Faktor IXa in Gegenwart von Calciumionen Faktor X schneller aktiviert als ohne Calciumionen. (J.Biol.Chem. 256, 3433-3442 (1981)), was naturgemäß unerwünscht ist. Es ist somit prinzipiell nicht möglich, bei den genannten Bestimmungsmethoden alle Komponenten zuerst zu einem Reagens zu mischen und erst danach der Probe zuzugeben.

Gemäß einem anderen Test ("COATEST[R]: Faktor VIIIc"; Hersteller: Kabi Vitrum) wird der Probe zunächst eine Mischung aus den bovinen Faktoren IXa und X mit Phospholipiden zugegeben, welche Mischung vor der Bestimmung frisch bereitet werden muß. Diese Mischung enthält zwar den Faktor X neben dem Faktor IXa, darf aber dafür keine Calciumionen enthalten, um die unerwünscht rasche Aktivierung des Faktors X nicht auszulösen. In diesem Fall muß daher das Calcium in einem eigenen Pipettierschritt der Probe zugefügt werden, und zwar nach einer Inkubation der mit der Mischung versetzten Probe; erst durch die Calcium-Zugabe wird die Reaktion gestartet. Nach einer weiteren Inkubation von 5 bis 10 min wird das chromogene Substrat zugegeben. Auch bei diesem Test muß somit eine fehlende Komponente zum Start der Reaktion in einem eigenen Schritt zugegeben werden; Komplexbildung und Aktivierung erfolgen zweistufig.

Eine Untersuching der intrinsischen Aktivierung von bovinem Faktor X durch die bovinen Faktoren IXa$\alpha$ und IXa$\beta$ ist in Arch. Biochem. Biophys. 268 (2) (1989), 485-501 beschrieben.

Die Erfindung stellt sich die Aufgabe, ein Reagens zur Verfügung zu stellen, das ein einfaches, mit möglichst wenigen Pipettierschritten durchführbares Bestimmungsverfahren der eingangs erwähnten Art ermöglicht. Das erfindungsgemäße Reagens soll sowohl zur Untersuchung von Körperflüssigkeiten auf ihre Faktor VIII-Aktivität, als auch zur automatisierten Untersuchung hochgereinigter Faktor VIII-Präparate eingesetzt werden können.

Das erfindungsgemäße Reagens zur Bestimmung von Faktor VIII-Aktivität ist dadurch gekennzeichnet, daß es Faktor IXa$\beta$, Faktor X, Calciumionen, Thrombin, Phosholipide und gegebenenfalls Faktor XIa und Faktor XIIa enthält.

Die Erfindung beruht auf der Erkenntnis, daß der Faktor IXa$\beta$ (in wässeriger Lösung) selbst in Gegenwart von Calciumionen den Faktor X während einer Zeitdauer von einigen Stunden nicht zu aktivieren vermag. Dadurch wird es möglich, alle für die Komplexbildung und Aktivierung notwendigen Bestandteile in einem einzigen Reagens zu vereinigen und somit dieses in einem einzigen Pipettierschritt mit der Probe zu mischen. Das erfindungsgemäße Reagens ist stundenlang stabil, kann also vor der Durchführung von Reihenuntersuchungen ohne Zeitdruck bereitet werden. Die Anwesenheit der zusätzlichen Gerinnungsfakto-ren XIa und XIIa wirkt auf den gebildeten Komplex stabilisierend.

Faktor IXa$\beta$ kann in bekannter Weise aus Humanplasma durch Behandeln mit Celite[R] (Hersteller: Johns-Manville Corp.) gewonnen werden.

Dem Reagens wird zweckmäßigerweise eine Substanz (z.B. ein Tetrapeptid) zugesetzt, um während der Testdauer eine Gerinnselbildung zu vermeiden. Es ist weiters zweckmäßig, dem Reagens einen Heparinneutralisator (z.B. Polybren) zuzusetzen, um die Bestimmung des Faktors VIII unabhängig vom Heparingehalt der zu untersuchenden Probe zu machen.

Die Erfindung betrifft auch ein Set bestehend aus zwei Reagenskomponenten A und B, zur Herstellung des erfindungsgemäßen Reagens, wobei die Reagenskomponente A die Faktoren IXaβ und X, Thrombin, Calciumionen und gegebenenfalls die Faktoren XIa und XIIa und die Reagenskomponente B Phospholipide enthält.

Die Erfindung betrifft weiters ein Set bestehend aus zwei Reagenskomponenten C und D, zur Herstellung des erfindungsgemäßen reagens, wobei die Reagenskomponente C die Faktoren IXaβ und X, Calciumionen und gegebenenfalls die Faktoren XIa und XIIa und die Reagenskomponente D Thrombin und Phospholipide enthält.

Eine bevorzugte Ausführungsform des erfindungsgemäßen Reagens besteht darin, daß Thrombin in einem Konzentrationsbereich von 0,01 und 2,0 E/ml vorliegt, Phospholipide in einem Bereich von 0,01 und 100 nmol/ml, Calciumionen in einem Bereich von 1 bis 50 $\mu$mol/ml, Faktor IXaβ in einem Bereich von 0,05 bis 5 E/ml, Faktor X in einem Bereich von 0,01 und 10 E/ml und gegebenenfalls die Faktoren XIa und XIIa jeweils in einem Bereich von 0,01 und 2,0 E/ml.

Zweckmäßigerweise sind die im erfindungsgemäßen Reagens enthaltenen Faktoren und das Thrombin humanen und nicht bovinen Ursprunges, wenn mit dem erfindungsgemäßen Reagens humane Faktor VIII-Aktivität bestimmt werden soll. Es ist ein Prinzip biochemischer Analytik, wo immer möglich, Gleiches mit Gleichem zu untersuchen, um Fehlerquellen auszuschließen, die erfahrungsgemäß bei Verwendung generisch verschiedenartiger Reaktionspartner auftreten. Dieser Nachteil kommt insbesondere dann zum Tragen, wenn die zu untersuchenden Proben eine hohe Faktor VIII-Aktivität besitzen, was z.B. bei der industriellen Qualitätskontrolle hochgereinigter Faktor VIII-Präparate von Bedeutung ist.

Die Verwendung boviner Gerinnungsfaktoren birgt auch die Gefahr, daß die Proteine nicht seuchenfrei sind, wodurch eine genaue und aufwendige Dokumentation der Herkunft der Faktoren erforderlich wird (Bescheinigung der Seuchenfreiheit der Rinder, einschließlich der Nichtinfektiosität des aus Rinderblut gewonnenen Rinderplasmas).

Gegebenenfalls sind die im erfindungsgemäßen Reagens enthaltenen Proteine einer Behandlung zur Inaktivlerung eventuell vorhandener infektiöser Agentien unterzogen. Derartige Inaktivierungsverfahren sind beispielsweise aus der EP-A 0 159 311 sowie der EP-A 0 050 061 und der EP-A 0 131 740 bekannt. Sie gewährleisten eine weitgehende Freiheit der Konzentrate von infektiösen Agentien.

Die Erfindung betrifft auch ein Verfahren zur Bestimmung von Faktor VIII-Aktivität in einer Probe durch Umsetzen mit dem erfindungsgemäßen Reagens, wobei zunächst in Abhängigkeit vom Faktor VIII-Gehalt aktivierter Faktor X (Faktor Xa) gebildet wird, und hernach der Faktor Xa quantitativ bestimmt wird. Diese Bestimmung kann beispielsweise so durchgeführt werden, daß der Faktor Xa zunächst mit einem chromogenen Substrat umgesetzt wird, um eine Substanz freizusetzen, welche spektrophotometrisch bestimmt wird.

Das erfindungsgemäße Verfahren besitzt eine Inkubationszeit von etwa fünf Minuten, wodurch sowohl ein manuelles Arbeiten als auch ein Arbeiten an Analyseautomaten, wie es für Reihenuntersuchungen charakteristisch ist, auf bequeme Weise ermöglicht wird.

Es hat sich weiters gezeigt, daß die photometrische Messung selbst während einer Zeitdauer bis zu vier Minuten erfolgen kann, da in diesem Zeitraum die Farbentwicklung (Freisetzung von p-Nitroanilin aus dem chromogenen Substrat) linear abläuft; das erfindungsgemäße Reagens besitzt somit die vorteilhafte Eigenschaft, während einer Zeitdauer von über vier Minuten die spektrophotometrisch bestimmbare Substanz mit konstanter Geschwindigkeit freizusetzen. Diese Zeitdauer ist ungewöhnlich lang, verglichen mit heute bekannten Reagenzien (beim Test "Dade[R] Faktor VIII Chromogen" (Hersteller: Fa. Baxter) beträgt sie nur etwa eine Minute).

Mit dem nachfolgenden Beispiel wird die Erfindung noch genauer beschrieben.

Herstellung des Reagens

(1) Faktor IXaβ-Lösung: 1 l Citratplasma wird mit 25 ml 1molarer CaCl$_2$-Lösung zur Herstellung von Humanserum versetzt und über Nacht bei Raumtemperatur stehen gelassen; das gebildete Gerinnsel wird ausgepreßt und verworfen. Das Serum wird filtriert und eingefroren.

Zur Adsorption von Faktor IXaβ aus Humanserum wird dieses bei 37°C aufgetaut, 0,5 mg/ml Sephadex[R] A 50 (Hersteller: Pharmacia) zugegeben, gerührt und dann filtriert. Das Gel wird anschließend in Puffer (2,5 % des Serumvolumens; 30 g/l NaCl, 4 g/l Na$_3$ Citrat.2H$_2$O, pH 7,0) eluiert. Das Eluat dient als Faktor IXaβ-Lösung.

(2) Faktor X-Lösung: Eine konzentrierte Lösung der Prothrombinkomplexfaktoren II, IX, X wird auf Dextransulfatsepharose aufgetragen und mit einem Ionenstärkegradienten (0,1 - 1 l) eluiert. Die Fraktio-

nen werden auf ihren Faktor X-Gehalt getestet. Jene Fraktionen, die Faktor X enthalten, werden gepoolt, durch Ultrafiltration aufkonzentriert und eingefroren. Konzentration: ca. 30 E Faktor X/ml.

(3) Celite-Eluat: Citratplasma wird mit 2 Gew.% Celit versetzt und unter Schütteln bei 37°C inkubiert. Anschließend wird 5 bis 10 Minuten bei 3000 Upm (Raumtemperatur) zentrifugiert. Der Überstand wird verworfen. Das Celite-Sediment wird mit physiologischer NaCl-Lösung dreimal gewaschen und dann mit 10 %iger NaCl-Lösung (1/4 Volumen des Ausgangsplasmas) eluiert und zentrifugiert. Der Überstand wird in Dialyseschläuche gefüllt und gegen das mindestens 20-fache Volumen einer physiologischen NaCl-Lösung über Nacht bei 4°C dialysiert und anschließend eingefroren.

Zur Herstellung der Reagenskomponente A werden folgende Komponenten gemischt:

| | |
|---|---|
| Imidazolpuffer (1,5 g/l Imidazol; 9 g/l NaCl; 0,1 % Albumin; pH 8,3) | 15,6 ml |
| Celite-Eluat | 1,05 ml |
| Faktor IXa$\beta$-Lösung | 1,2 ml |
| Faktor X-Lösung | 0,3 ml |
| 20 % Humanalbumin | 0,9 ml |
| CaCl$_2$ 25mmol/l | 18,0 ml |
| Thrombin | 0,12 ml |

Diese Mischung wird bei 4°C über Nacht gegen einen Puffer (1,36 g/l Imidazol, 2,34 g/l NaCl, 1,47 g/l Natriumcitrat, 13 ml 1 mol/l CaCl$_2$) dialysiert und anschließend filtriert. Gerinnungsaktives Phospholipidkonzentrat wird 1:200 mit Dialysepuffer verdünnt und 1 Teil dieser Lösung (Reagenskomponente B) mit 3 Teilen der filtrierten Mischung versetzt. Nach Zusatz von 0,19 % Tween 20 (Polyoxyethylensorbitanmonolaureat) und 5 mg Tetrapeptid AcOH-Gly-Pro-Arg-Pro-OH wird das Reagens zu je 2 ml abgefüllt.

Bestimmung von Faktor VIII in einer Probe:

Zunächst wird 1 Teil einer Faktor Xa-Substratlösung (CH$_3$OCO-D-CHA-Gly-Arg-pNA.AcOH, 4 mmol/l) mit 2 Teilen eines Reaktionspuffers (6,06 g/l Tris; 3,03 g/l EDTA; 25 g/l NaCl; 4 ATU/ml Hirudin; pH 8,2) gemischt (Substratpuffergemisch).

Pipettierschema:

50 $\mu$l einer Faktor VIII-hältigen Probe und 250 $\mu$l Reagens werden 5 min bei 37°C inkubiert und mit 300 $\mu$l Substratpuffergemisch versetzt.

Die spektrophotometrische Bestimmung des Faktor VIII erfolgt in an sich bekannter Weise, wobei die Messung nach 3 minütigem Halten der Lösung (37°C) bei 405 nm durchgeführt wird. Die Erstellung der Bezugskurve erfolgt ebenso nach bekannter Weise, wobei von einem lyophilisierten Normalplasmapool ausgegangen wird, der nach Lösen mit Aqua dest. 1 I.E. Faktor VIII/ml enthalten soll.

Es hat sich gezeigt, daß die erfindungsgemäße Bestimmungsmethode einen linearen Zusammenhang zwischen der Extinktionszunahme pro Zeiteinheit und dem Faktor VIII-Gehalt im Konzentrationsbereich zwischen 2 I.E./ml und 0,002 I.E./ml zeigt.

Die erfindungsgemäße Bestimmungsmethode weist eine hohe Sensitivität auf und eignet sich aus diesem Grund auch zur indirekten Bestimmung von Proteinen, die mit dem Faktor VIII (aktiviert oder nicht aktiviert) reagieren. So kann z.B. der Gehalt an aktiviertem Protein C und seinem Kofaktor (Protein S) oder Faktor VIII-Inhibitor indirekt bestimmt werden, indem der Probe eine bestimmte Menge an Faktor VIII (aktiviert oder nicht aktiviert) zugegeben wird, worauf der vom aktivierten Protein C und seinem Kofaktor bzw. Faktor VIII-Inhibitor nicht inhibierte Anteil des Faktors VIII bestimmt wird.

Das erfindungsgemäße Verfahren kann somit gleichfalls zur indirekten Bestimmung der Faktor VIII-inhibierenden Aktivität angewandt werden.

Bestimmung von aktiviertem Protein C (aPC)

Reagenskomponente A und B werden, wie bei der Herstellung des Reagens bereits beschrieben, verwendet.

75 $\mu$l Faktor VIII-Lösung (5 E/ml) werden durch Zugabe von 35 $\mu$l Thrombin-Lösung (0,5 E/ml) und 5 min Inkubation bei 37°C aktiviert. 50 $\mu$l der Probelösung mit unterschiedlichem Gehalt an aPC (0,03; 0,10; 0,30; 1,00 E/ml) werden mit 100 $\mu$l Inkubationsgemisch in Gegenwart von 200 $\mu$l Puffergemisch (5 ml Tris-Puffer, pH 7,4; 100 $\mu$l Hirudin und 25 $\mu$l Phospholipid-Reagenskomponente B) versetzt und 2 min bei 37°C inkubiert. Nach Zusatz von 200 $\mu$l der Reagenskomponente A zu 100 $\mu$l der inkubierten Probelösung und anschließender Inkubation (5 min bei 37°C) wird der von aPC nicht inhibierte Anteil an Faktor VIII photometrisch bestimmt. Die Probe wird mit dem bereits beschriebenen Substratpuffergemisch versetzt. Die Extinktion bei 405 nm wird 3 min lang bei 37°C gemessen. Es besteht ein linearer Zusammenhang zwischen der Zunahme der Extinktion mit der Zeit (dE/min) und der Konzentration an aPC in der Probelösung.

| Konzentration aPC (E/ml) | dE/min |
|---|---|
| 0 | 0,245 |
| 0,03 | 0,243 |
| 0,10 | 0,239 |
| 1,00 | 0,119 |

## Patentansprüche

1. Reagens zur Bestimmung von Faktor VIII-Aktivität, dadurch gekennzeichnet, daß es Faktor IXa$\beta$, Faktor X, Calciumionen, Thrombin, Phospholipide und gegebenenfalls Faktor XIa und Faktor XIIa enthält.

2. Reagenskomponente zur Herstellung eines Reagens nach Anspruch 1, dadurch gekennzeichnet, daß sie Faktor IXa$\beta$, Faktor X und Calciumionen enthält.

3. Set bestehend aus zwei Reagenskomponenten A und B, zur Herstellung eines Reagens nach Anspruch 1, wobei die Reagenskomponente A die Faktoren IXa$\beta$ und X, Thrombin, Calciumionen und gegebenenfalls die Faktoren XIa und XIIa und die Reagenskomponente B Phospholipide enthält.

4. Set bestehend aus zwei Reagenskomponenten C und D, zur Herstellung eines Reagens nach Anspruch 1, wobei die Reagenskomponente C die Faktoren IXa$\beta$ und X, Calciumionen und gegebenenfalls die Faktoren XIa und XIIa und die Reagenskomponente D Thrombin und Phospholipide enthält.

5. Reagens nach Anspruch 1, dadurch gekennzeichnet, daß Thrombin in einem Konzentrationsbereich von 0,01 und 2,0 E/ml vorliegt, Phospholipide in einem Bereich von 0,01 und 100 nmol/ml, Calciumionen in einem Bereich von 1 bis 50 $\mu$mol/ml, Faktor IXaß in einem Bereich von 0,05 bis 5 E/ml, Faktor X in einem Bereich von 0,01 und 10 E/ml und gegebenenfalls die Faktoren XIa und XIIa jeweils in einem Bereich von 0,01 und 2,0 E/ml.

6. Reagens nach einem der Ansprüche 1 oder 5 oder Reagenskomponente nach Anspruch 2 oder Set nach einem der Ansprüche 3 oder 4, dadurch gekennzeichnet, daß die enthaltenen Faktoren und das Thrombin humanen Ursprunges sind und gegebenenfalls einer Behandlung zur Inaktivierung eventuell vorhandener infektiöser Agentien unterzogen sind.

7. Verfahren zur Bestimmung von Faktor VIII-Aktivität in einer Probe durch Umsetzen mit einem Reagens nach Anspruch 1, dadurch gekennzeichnet, daß zunächst in Abhängigkeit des Faktor VIII-Gehaltes aktivierter Faktor X (Faktor Xa) gebildet wird, und hernach der Faktor Xa quantitativ bestimmt wird.

8. Verfahren zur Bestimmung von Proteinen, die mit Faktor VIII reagieren, insbesondere von Protein C und seinem Cofaktor Protein S, von Faktor VIII-Antikörpern bzw. von Faktor VIII-inhibierenden Proteinen, dadurch gekennzeichnet, daß nach Zugabe einer bestimmten überschüssigen Menge an Faktor VIII zur Probe die verbleibende Menge des Faktors VIII mit einem Reagens nach Anspruch 1 umgesetzt und der durch nicht inhibierten Faktor VIII aktivierte Faktor X bestimmt wird.

EP 0 496 723 B1

**9.** Verfahren nach Anspruch 8, dadurch gekennzeichnet, daß das Reagens eine bestimmte zusätzliche Menge Faktor VIII enthält.

**Claims**

**1.** A reagent for determining factor VIII-activity, characterised in that it comprises factor IXa$\beta$, factor X, calcium ions, thrombin, phospholipids and possibly factor XIa and factor XIIa.

**2.** A reagent component for producing a reagent according to claim 1, characterised in that it contains factor IXa$\beta$, thrombin, factor X and calcium ions.

**3.** A kit comprised of two reagent components A and B, for producing a reagent according to claim 1, wherein the reagent component A contains the factors IXa$\beta$ and X, thrombin, calcium ions and possibly factors XIa and XIIa, and the reagent component B contains phospholipids.

**4.** A kit comprised of two reagent components C and D, for producing a reagent according to claim 1, wherein the reagent component C contains the factors IXa$\beta$ and X, calcium ions and possibly the factors XIa and XIIa, and the reagent component D contains thrombin and phospholipids.

**5.** A reagent according to claim 1, characterised in that thrombin is present in a concentration range of from 0.01 to 2.0 U/ml, phospholipids are present in a range of from 0.01 to 100 nmol/ml, calcium ions are present in a range of from 1 to 50 $\mu$mol/ml, factor IXa$\beta$ is present in a range of from 0.05 to 5 U/ml, factor X is present in a range of from 0.01 to 10 U/ml, and, possibly, the factors XIa and XIIa are each present in a range of from 0.01 to 2.0 U/ml.

**6.** A reagent according to any one of claims 1 or 5, or a reagent component according to claim 2, or a kit acccording to any one of claims 3 or 4, characterised in that the factors contained therein and the thrombin are of human origin and, if desired, are subjected to a treatment for inactivation of infectious agents possibly present.

**7.** A method of determining the factor VIII activity in a sample by reacting with a reagent according to claim 1, characterised in that at first activated factor X (factor Xa) is formed in dependence on the factor VIII content, and subsequently factor Xa is quantitatively determined.

**8.** A method of determining proteins reacting with factor VIII, in particular protein C and its co-factor protein S, of factor VIII antibodies and of proteins inhibiting factor VIII, characterised in that after the addition of a predetermined excess amount of factor VIII to the sample, the remaining amount of factor VIII is reacted with a reagent according to claim 1 and the factor X activated by non-inhibited factor VIII is determined.

**9.** A method according to claim 8, characterised in that the reagent contains a predetermined additional amount of factor VIII.

**Revendications**

**1.** Réactif destiné à la détermination de l'activité facteur VIII, caractérisé en ce qu'il contient le facteur IXa$\beta$, le facteur X, des ions calcium, de la thrombine, des phospholipides et le cas échéant le facteur XIa et le facteur XIIa.

**2.** Composant de réactif destiné à la préparation d'un réactif selon la revendication 1, caractérisé en ce qu'il contient le facteur IXa$\beta$, de la thrombine, le facteur X et des ions calcium.

**3.** Ensemble constitué de deux composants de réactif A et B, destiné à la préparation d'un réactif selon la revendication 1, le composant de réactif A contenant les facteurs IXa$\beta$ et X, de la thrombine, des ions calcium et le cas échéant les facteurs XIa et XIIa et le composant de réactif B des phospholipides.

**4.** Ensemble constitué de deux composants de réactif C et D, destiné à la préparation d'un réactif selon la revendication 1, le composant de réactif C contenant les facteurs IXa$\beta$ et X, des ions calcium et le cas

6

échéant les facteurs XIa et XIIa et le composant de réactif D de la thrombine et des phospholipides.

5. Réactif selon la revendication 1, caractérisé en ce que la thrombine est présente dans une plage de concentrations de 0,01 à 2,0 U/ml, les phospholipides dans une plage de 0,01 à 100 nmol/ml, les ions calcium dans une plage de 1 à 50 $\mu$mol/ml, le facteur IXa$\beta$ dans une plage de 0,05 à 5 U/ml, le facteur X dans une plage de 0,01 à 10 U/ml et le cas échéant les facteurs XIa et XIIa dans une plage respective de 0,01 à 2,0 U/ml.

6. Réactif selon une des revendications 1 ou 5 ou composant de réactif selon la revendication 2 ou ensemble selon une des revendications 3 et 4, caractérisé en ce que les facteurs contenus et la thrombine sont d'origine humaine et sont soumis le cas échéant à un traitement d'inactivation des agents infectieux éventuellement présents.

7. Procédé de détermination de l'activité facteur VIII d'un échantillon par mise à réagir avec un réactif selon la revendication 1, caractérisé en ce qu'on forme d'abord du facteur X activé (facteur Xa) en fonction de la teneur en facteur XIII et qu'on détermine ensuite quantitativement le facteur Xa.

8. Procédé de détermination de protéines réagissant avec le facteur VIII, notamment la protéine C et son cofacteur la protéine S, d'anticorps du facteur VIII et de protéines inhibant le facteur VIII, caractérisé en ce qu'après l'addition d'une quantité déterminée en excès de facteur VIII à l'échantillon, on met à réagir la quantité résiduelle de facteur VIII avec un réactif selon la revendication 1 et qu'on détermine le facteur X activé par le facteur VIII non inhibé.

9. Procédé selon la revendication 8, caractérisé en ce que le réactif contient une quantité additionnelle déterminée de facteur VIII.